# EUROPEAN PATENT APPLICATION

(11) **EP 1 717 307 A1**
(43) Date of publication of application: **02.11.2006**
(21) Application number: 04748263.3
(22) Date of filing: 30.07.2004
(51) Int. Cl.: C12M 1/38

(54) **TEMPERATURE CONTROL DEVICE**

(30) Priority: 14.01.2004 JP 2004006988
(71) Applicant: DAIKIN INDUSTRIES, LTD., Osaka-shi, Osaka 530-8323 (JP)
(72) Inventor: MIYAHARA, Seiichiro, c/o Daikin Industries, Ltd., Tukuba-shi, Ibaraki 305-0481 (JP)
(74) Representative: Goddar, Heinz J.
(86) International application number: PCT/JP2004/011292
(87) International publication number: WO 2005/068607

(57) **Abstract**

The present invention relates to a temperature control device, and has an object to accurately control temperatures of microorganisms or cells when for example applied to culture and so on of the microorganisms or cells, for example. The temperature control device includes a plurality of cell parts (2) holding microorganisms or cells, a heater (1), and a cooling unit (7). The heater (1) is provided around the cell parts (2) to heat the cell parts (2). The cooling unit (7) includes a cooling fan (71), a cooling fin (72), an aluminum conduction block (73), a Peltier element (74), a radiating fin (75), and a radiating fan (76). Air (701) is supplied to the cooling fin (72) via the cooling fan (71). The air (701) is cooled in the cooling fin (72) because heat is moved from the air (701) to the cooling fin (72). Air (702) having been cooled is sent to the cell parts (2) to cool the cell parts (2). Temperatures inside the cell parts are controlled by controlling operations of the heater (1) and the cooling unit (7).

## Description

### Technical Field

The present invention relates to temperature control devices, and can be applied to culture and so on of microorganisms or cells, for example.

### Background Art

A pace of culture and so on of microorganisms or cells is sensitive to a temperature inside a cell part. It is thus desirable to accurately control a temperature inside a cell part. Therefore, conventional culture devices include a plurality of cell parts, and a heater for heating the cell parts. The cell parts hold microorganisms or cells.

Non-patent document 1 introduces a culture device for measuring the number of microorganisms or cells from a current flowing through a culture medium while culturing the microorganisms or cells.

Non-patent document 1; "Food Bacteria Inspection System DOX-60F/30F", [online], DAIKIN INDUSTRIES, LTD., [November 20, 2003], Internet <URL:http://www.del.co.jp/products/dox>;

### Disclosure of Invention

In the conventional culture devices, a target value for a temperature inside the cell parts (hereafter called an "intra-cell temperature") has been employed as a target value for a temperature of the heater (hereafter called a "heater temperature"). The target value for the intra-cell temperature is a desirable temperature for culturing the microorganisms or cells, for example.

The heater includes a heater line and a thermal conductor. Accordingly, heat is conducted from the heater line to the microorganisms or cells inside the cell parts via the thermal conductor and the cell parts. This may cause the intra-cell temperature actually measured to be different from the target value for the heater temperature. Further, the intra-cell temperature may vary under the influence of an ambient temperature of an environment in which the culture device is installed.

In addition, the intra-cell temperature has been controlled only by the heater. The result has been that it takes time to reduce the intra-cell temperature to a desirable temperature.

The present invention has been made in view of the circumstances described above, and accurately controls temperatures of microorganisms or cells.

A temperature control device according to claim 1 of the present invention includes a plurality of cell parts (2) holding microorganisms or cells; and a heater (1) and a cooling unit (7) making control of temperatures inside the cell parts, and the control is corrected by using an ambient temperature (T1).

According to the temperature control device of claim 1 of the present invention, where the intra-cell temperature is controlled by using the heater and the cooling unit, the temperatures of the microorganisms or cells can be controlled with stability to time. Further, with consideration being given to the ambient temperature at the time of the control, the influence of the ambient temperature upon the intra-cell temperature is small.

A temperature control device according to claim 2 of the present invention is the temperature control device according to claim 1, wherein the heater (1) includes: a first heater line (11; 11, 12) and a second heater line (14; 13); a plurality of first thermal conductors (31; 31, 32) provided for the first heater line; and a plurality of second thermal conductors (32, 33; 33) provided for the second heater line.

According to the temperature control device of claim 2 of the present invention, where the first thermal conductor and the second thermal conductor are divided to be provided for the first and second heater lines, respectively, the weight and size of the heater are reduced further than when a single-piece thermal conductor is provided.

A temperature control device according to claim 3 of the present invention is the temperature control device according to claim 1, wherein the heater (1) includes: a first heater line (11) and a second heater line (14); a plurality of first thermal conductors (31) provided for the first heater line; and a plurality of second thermal conductors (32, 33) provided for the second heater line, and the first thermal conductor and the second thermal conductor are controlled to different temperatures from each other.

According to the temperature control device of claim 3 of the present invention, temperatures near the thermal conductors can be varied for each heater, which allows concurrent culture and so on of microorganisms or cells under different conditions.

A temperature control device according to claim 4 of the present invention is the temperature control device according to claim 1, wherein the heater (1) includes: a first heater line (11, 12) and a second heater line (13); a plurality of first thermal conductors (31, 32) provided for the first heater line; a plurality of second thermal conductors (33) provided for the second heater line; a first thermometer (41, 42) provided for one of the first thermal conductors; and a second thermometer (43) provided for one of the second thermal conductors, the first thermal conductors being equal in thermal capacity, the second thermal conductors being equal in thermal capacity, and the first thermal conductors and the second thermal conductors being different from each other in thermal capacity.

According to the temperature control device of claim 4 of the present invention, the first thermal conductors and the second thermal conductors are different in thermal capacity. This enhances a degree of freedom to arrange the first heater line and the second heater line to heat the plurality of cell parts uniformly. On the other hand, it is assumed that a temperature of the first thermal conductor measured by the first thermometer is almost equal to a temperature of the first thermal conductor not provided with the first thermometer. It is also assumed that a temperature of the second thermal conductor measured by the second thermometer is almost equal to a temperature of the second thermal conductor not provided with the second thermometer. Accordingly, when controlling the first heater line and the second heater line by using the first thermometer and the second thermometer, temperatures near the first and second thermal conductors can be made almost equal. In short, the overall temperature of the heater can be made almost uniform. The result is that the temperature to which a pace of culture and so on of microorganisms or cells is sensitive can be controlled accurately.

A temperature control device according to claim 5 of the present invention is the temperature control device according to claim 1, further including: a thermometer (45) measuring the ambient temperature (T1); a storage unit (5) storing calibration data; and a control unit (6) setting a target value (T0) for the temperatures inside the cell parts, and controlling the heater (1) and the cooling unit (7) with a second target value (T2) that is obtained based on the target value (T0) and the calibration data in accordance with the ambient temperature.

According to the temperature control device of claim 5 of the present invention, the second target value is set for each ambient temperature. The intra-cell temperature thus reaches the target value accurately.

A temperature control device according to claim 6 of the present invention is the temperature control device according to claim 5, wherein the heater (1) includes: a first heater line (11; 11, 12) and a second heater line (14; 13); a plurality of first thermal conductors (31; 31, 32) provided for the first heater line; and a plurality of second thermal conductors (32, 33; 33) provided for the second heater line.

According to the temperature control device of claim 6 of the present invention, where the first thermal conductor and the second thermal conductor are divided to be provided for the first and second heater lines, respectively, the weight and size of the heater are reduced further than when a single-piece thermal conductor is provided.

A temperature control device according to claim 7 of the present invention is the temperature control device according to claim 5, wherein the heater (1) includes: a first heater line (11) and a second heater line (14); a plurality of first thermal conductors (31) provided for the first heater line; and a plurality of second thermal conductors (32, 33) provided for the second heater line, and the first thermal conductor and the second thermal conductor are controlled to different temperatures from each other.

According to the temperature control device of claim 7 of the present invention, temperatures near the thermal conductors can be varied for each heater, which allows concurrent culture and so on of microorganisms or cells under different conditions.

A temperature control device according to claim 8 of the present invention is the temperature control device according to claim 5, wherein the heater (1) includes: a first heater line (11, 12) and a second heater line (13); a plurality of first thermal conductors (31, 32) provided for the first heater line; a plurality of second thermal conductors (33) provided for the second heater line; a first thermometer (41, 42) provided for one of the first thermal conductors; and a second thermometer (43) provided for one of the second thermal conductors, the first thermal conductors being equal in thermal capacity, the second thermal conductors being equal in thermal capacity, and the first thermal conductors and the second thermal conductors being different from each other in thermal capacity.

According to the temperature control device of claim 8 of the present invention, the first thermal conductors and the second thermal conductors are different in thermal capacity. This enhances a degree of freedom to arrange the first heater line and the second heater line to heat the plurality of cell parts uniformly. On the other hand, it is assumed that a temperature of the first thermal conductor measured by the first thermometer is almost equal to a temperature of the first thermal conductor not provided with the first thermometer. It is also assumed that a temperature of the second thermal conductor measured by the second thermometer is almost equal to a temperature of the second thermal conductor not provided with the second thermometer. Accordingly, when controlling the first heater line and the second heater line by using the first thermometer and the second thermometer, temperatures near the first and second thermal conductors can be made almost equal. In short, the overall temperature of the heater can be made almost uniform. The result is that the temperature to which a pace of culture and so on of microorganisms or cells is sensitive can be controlled accurately.

A temperature control device according to claim 9 of the present invention is the temperature control device according to claim 1, further including: a thermometer (45) measuring the ambient temperature (T1); a control unit (6) setting a target value (T0) for the temperatures inside the cell parts; and a calculation unit, wherein the calculation unit calculates a second target value (T2) from the ambient temperature and the target value (T0), and the control unit controls the heater (1) and the cooling unit (7) with the second target value (T2).

According to the temperature control device of claim 9 of the present invention, the second target value is set for each ambient temperature. The intra-cell temperature thus reaches the target value accurately.

A temperature control device according to claim 10 of the present invention is the temperature control device according to claim 9, wherein the heater (1) includes: a first heater line (11; 11, 12) and a second heater line (14; 13); a plurality of first thermal conductors (31; 31, 32) provided for the first heater line; and a plurality of second thermal conductors (32, 33; 33) provided for the second heater line.

According to the temperature control device of claim 10 of the present invention, where the first thermal conductor and the second thermal conductor are divided to be provided for the first and second heater lines, respectively, the weight and size of the heater are reduced further than when a single-piece thermal conductor is provided.

A temperature control device according to claim 11 of the present invention is the temperature control device according to claim 9, wherein the heater (1) includes: a first heater line (11) and a second heater line (14); a plurality of first thermal conductors (31) provided for the first heater line; and a plurality of second thermal conductors (32, 33) provided for the second heater line, and the first thermal conductor and the second thermal conductor are controlled to different temperatures from each other.

According to the temperature control device of claim 11 of the present invention, temperatures near the thermal conductors can be varied for each heater, which allows concurrent culture and so on of microorganisms or cells under different conditions.

A temperature control device according to claim 12 of the present invention is the temperature control device according to claim 9, wherein the heater (1) includes: a first heater line (11, 12) and a second heater line (13); a plurality of first thermal conductors (31, 32) provided for the first heater line; a plurality of second thermal conductors (33) provided for the second heater line; a first thermometer (41, 42) provided for one of the first thermal conductors; and a second thermometer (43) provided for one of the second thermal conductors, the first thermal conductors being equal in thermal capacity, the second thermal conductors being equal in thermal capacity, and the first thermal conductors and the second thermal conductors being different from each other in thermal capacity.

According to the temperature control device of claim 12 of the present invention, the first thermal conductors and the second thermal conductors are different in thermal capacity. This enhances a degree of freedom to arrange the first heater line and the second heater line to heat the plurality of cell parts uniformly. On the other hand, it is assumed that a temperature of the first thermal conductor measured by the first thermometer is almost equal to a temperature of the first thermal conductor not provided with the first thermometer. It is also assumed that a temperature of the second thermal conductor measured by the second thermometer is almost equal to a temperature of the second thermal conductor not provided with the second thermometer. Accordingly, when controlling the first heater line and the second heater line by using the first thermometer and the second thermometer, temperatures near the first and second thermal conductors can be made almost equal. In short, the overall temperature of the heater can be made almost uniform. The result is that the temperature to which a pace of culture and so on of microorganisms or cells is sensitive can be controlled accurately.

A temperature control device according to claim 13 of the present invention is the temperature control device according to claim 12, wherein the second heater line (13) is provided on an outer edge side of the heater than the first heater line (11, 12) is, each of the first thermal conductors (31, 32) includes a pair of heat blocks (3) provided on both sides of the first heater line, and each of the second thermal conductors (33) includes one heat block (3) provided for the second heater line on the side of the first heater line.

According to the temperature control device of claim 13 of the present invention, the number of heat blocks is reduced. Accordingly, the weight and size of the heater are reduced.

A temperature control device according to claim 14 of the present invention is the temperature control device according to any one of claims 1 to 13, further including a sensor for each of the cell parts (2), the sensor measuring a measurement value that varies depending on metabolism of the microorganisms or cells.

According to the temperature control device of claim 14 of the present invention, the number of microorganisms or cells is estimated.

These and other objects, features, aspects and advantages of the present invention will become more apparent from the following detailed description of the present invention when taken in conjunction with the accompanying drawings.

### Brief Description of Drawings

Fig. 1 is a perspective view schematically showing a temperature control device to be described in a first preferred embodiment.
Figs. 2 are cross-sectional views taken along a position A-A and a position B-B of the temperature control device shown in Fig. 1.
Figs. 3 and 4 are block diagrams schematically showing control-correcting functions based on an ambient temperature.
Fig. 5 is a plan view schematically showing a heater to be described in a second preferred embodiment.
Figs. 6 and 7 are plan views schematically showing heaters to be described in a third preferred embodiment.

### Best Modes for Carrying Out the Invention

### 1. First Preferred Embodiment

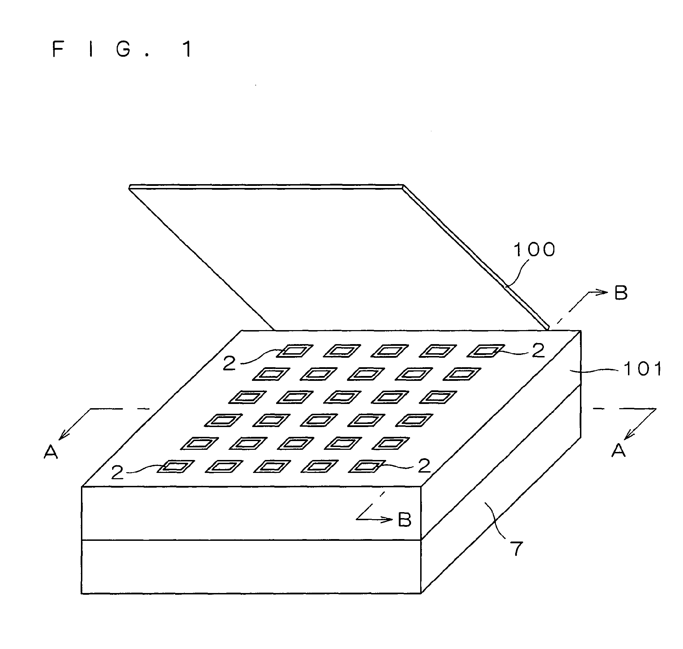

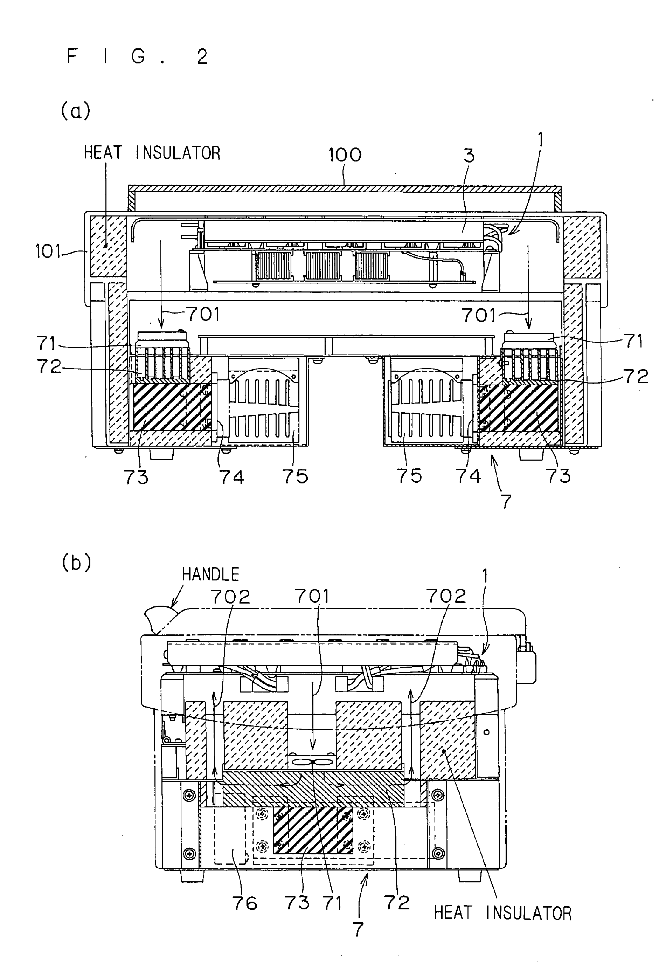

Fig. 1 is a perspective view schematically showing a temperature control device according to a first preferred embodiment. Figs. 2(a) and (b) are cross-sectional views taken along a position A-A and a position B-B of a bacteria culture device shown in Fig. 1. The temperature control device includes a plurality of cell parts 2, a heater 1, and a cooling unit 7. The plurality of cell parts 2 hold microorganisms or cells. The heater 1 and the cooling unit 7 are both used to control an intra-cell temperature.

A case 101 is provided with a plurality of holes. The cell parts 2 each have an opening and a lid. The microorganisms or bacteria are put into the opening. The lid is provided to close the opening. The cell parts 2 are housed in the holes of the case 101 so that the opening side of the cell parts 2 is positioned on the surface side of the case 101. The temperature control device is further provided with a cover 100 in Fig. 1, which prevents foreign matters such as dust from entering the holes of the case 101.

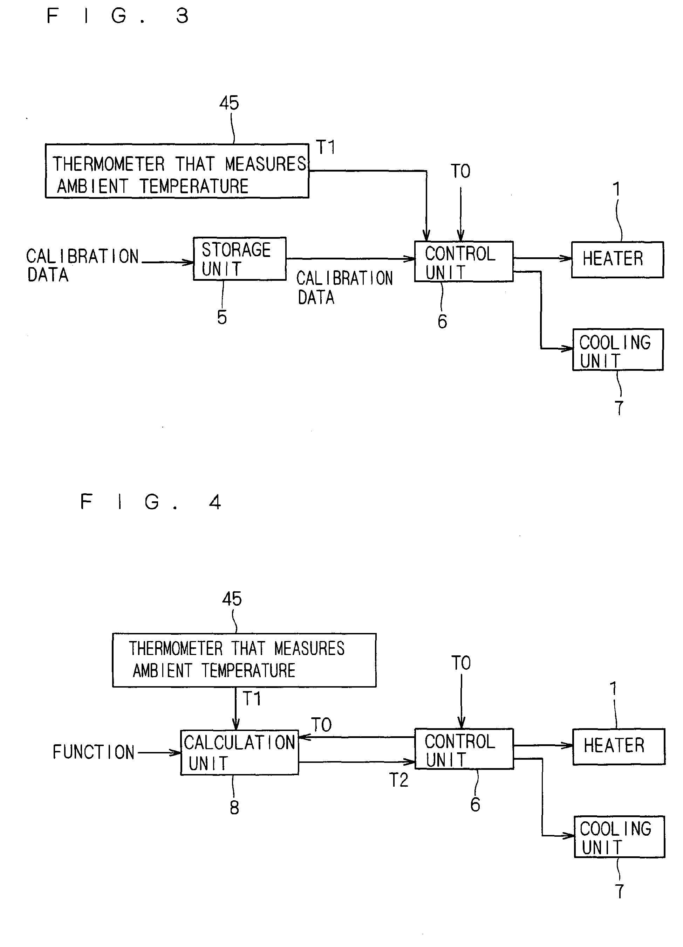

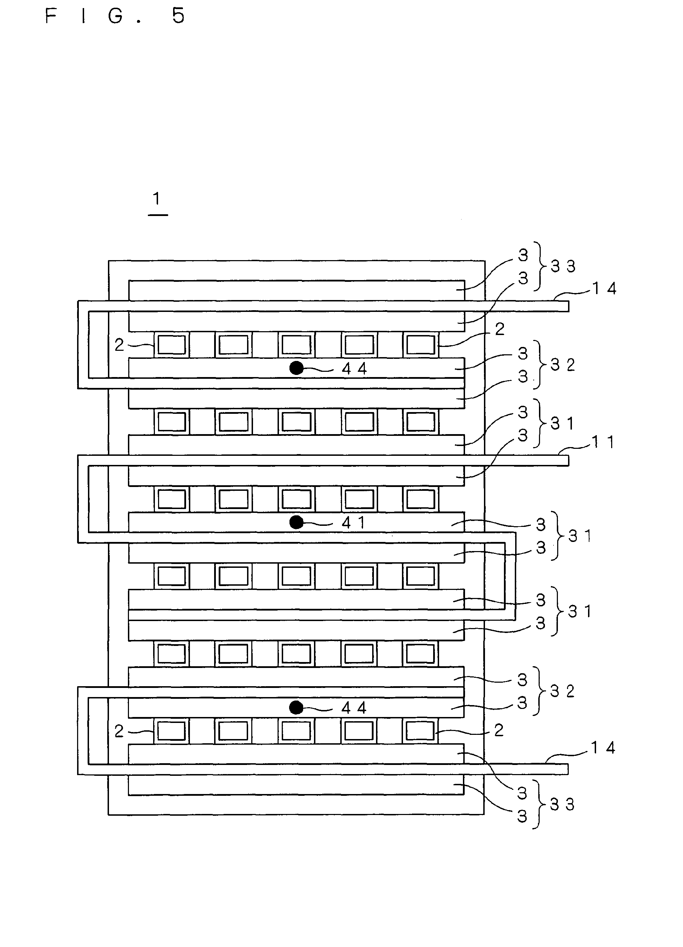

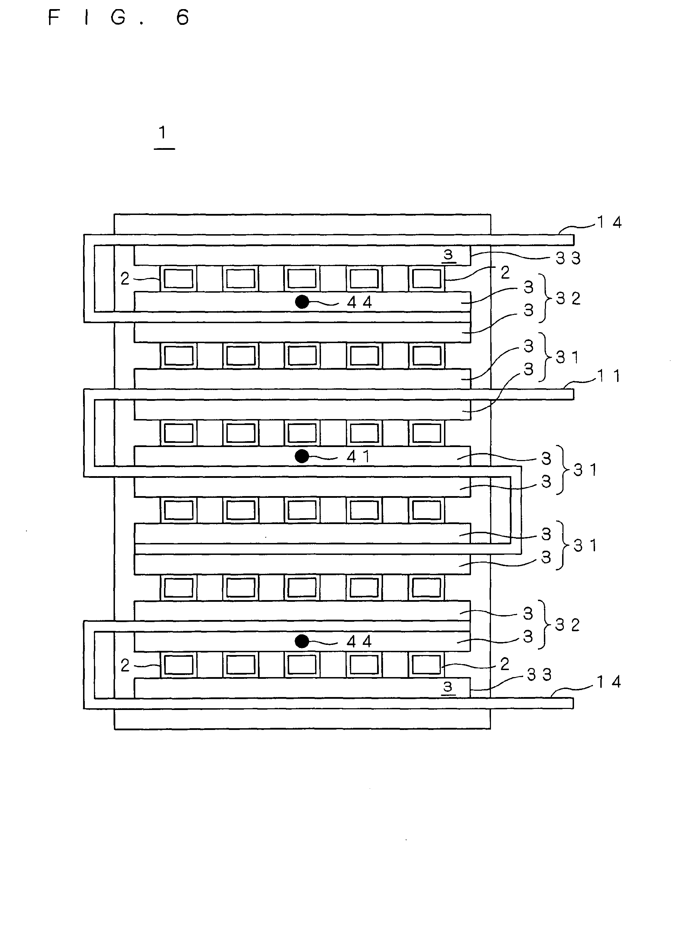

Heaters 1 shown in Figs. 5 to 7 to be described later, for example, can be employed as the heater 1. The heater 1 is provided around the cell parts 2 to heat the cell parts 2.

The cooling unit 7 includes a cooling fan 71, a cooling fin 72, an aluminum conduction block 73, a Peltier element 74, a radiating fin 75 and a radiating fan 76.

Air 701 is supplied to the cooling fin 72 via the cooling fan 71. The cooling fin 72 absorbs heat from the air 701 to cool the air 701. Air 702 having been cooled is sent to the cell parts 2 to cool the cell parts 2. The flow of the air 701 and 702 is shown by the arrows in Figs. 2. In addition to the flow of the air 701 and 702 in the direction shown by the arrows in Fig. 2, the air 702 may be supplied to the cooling fan 71 via the cooling fin 72, to send the air 701 having been cooled to the cell parts 2.

The heat obtained by the cooling fin 72 is supplied to the aluminum conduction block 73. The Peltier element 74 moves the heat from the aluminum conduction block 73 side to the radiating fin 75 side. The heat having been moved to the radiating fin 75 is released to the outside by the radiating fan 76.

The temperature control device corrects, based on an ambient temperature, control exercised by the heater 1 and the cooling unit 7, respectively. This function possessed by the temperature control device is shown as a block diagram in Fig. 3, for example. The temperature control device further includes a thermometer 45, a storage unit 5 and a control unit 6. The thermometer 45 measures an ambient temperature T1. The storage unit 5 stores calibration data. The control unit 6 controls the heater 1 and the cooling unit 7, respectively.

The calibration data is obtained as follows, for example. An intra-cell temperature with respect to a target value for a heater temperature is measured in advance for each ambient temperature. The target value for the heater temperature is set by the control unit 6, for example. Then, the relationship between the target value for the heater temperature and the intra-cell temperature is presented in the form of table for each ambient temperature, to be employed as the calibration data.

Upon being supplied with a target value T0 for the intra-cell temperature, the control unit 6 is further supplied with the ambient temperature T1 and the calibration data. The control unit 6 obtains, based on the calibration data corresponding to the ambient temperature T1, a target value T2 for the heater temperature where the intra-cell temperature becomes the target value T0. The control unit 6 then controls the heater 1 and the cooling unit 7 with the target value T2 for the heater temperature.

Assuming that the target value T2 for the heater temperature is a second target value with respect to the target value T0 for the intra-cell temperature, the above descriptions are understood as follows. That is, the target value T0 for the intra-cell temperature is set, and the control unit 6 controls the heater 1 with the second target value that is obtained based on the target value T0 for the intra-cell temperature and the calibration data in accordance with the ambient temperature T1.

The above function possessed by the temperature control device may alternatively be organized by a block diagram shown in Fig. 4. The temperature control device includes a calculation unit 8 in place of the storage unit 5 shown in Fig. 3. The calculation unit 8 performs calculations based on a predetermined function.

The predetermined function is obtained as follows, for example. An intra-cell temperature with respect to a target value for a heater temperature is measured in advance for each ambient temperature. Then, based on resultant data, the relationship among the target value for the heater temperature, the ambient temperature, and the intra-cell temperature is presented in the form of function, to be employed as the predetermined function.

First, the control unit 6 is supplied with a target value T0 for the intra-cell temperature, and supplies the same to the calculation unit 8. The calculation unit 8 is further supplied with the ambient temperature T1 from the thermometer 45. Based on the predetermined function using the target value T0 for the intra-cell temperature and the ambient temperature T1, the calculation unit 8 obtains a target value T2 for the heater temperature where the intra-cell temperature becomes the target value T0. The target value T2 for the heater temperature is then supplied to the control unit 6. The control unit 6 controls the heater 1 and the cooling unit 7 with the target value T2 for the heater temperature. The target value T2 for the heater temperature can be regarded as a second target value with respect to the target value T0 for the intra-cell temperature.

According to the temperature control device described above, where the intra-cell temperature is controlled by using the heater 1 and the cooling unit 7, the temperatures of the microorganisms or cells can be controlled with stability to time. For instance, the heater 1 is activated when the intra-cell temperature is lower than a target value, and the cooling unit 7 is activated when the intra-cell temperature is higher than a target value, so that the intra-cell temperature can be stabilized near the target value. Alternatively, both of the heater 1 and the cooling unit 7 may be activated at the same time.

Moreover, the temperature control device including the storage unit 5 or the calculation unit 8 allows the second target value T2 to be set for each ambient temperature T1. The intra-cell temperature thus reaches the target value T0 accurately. Namely, the intra-cell temperature is controlled in consideration of the ambient temperature, making the influence of the ambient temperature upon the intra-cell temperature small.

The functions shown as the block diagrams in Figs. 3 and 4 can be organized by employing conventional techniques. For instance, a microcomputer can be employed as the control unit 6.

The temperature control device described above can be applied to other purposes than culture of microorganisms or cells. For instance, with microorganisms or cells as a medium, the amount, influence and the like of a chemical substrate can be measured by utilizing their respiration activities. Another application would be when microorganisms or cells die out.

### 2. Second Preferred Embodiment

Fig. 5 schematically shows a heater 1 according to a second preferred embodiment. Like the heater 1 according to the first preferred embodiment, this heater 1 can be applied to the temperature control device shown in the first preferred embodiment. The heater 1 includes a heater line 11 and two heater lines 14. The heater lines 14 are provided on an outer edge side of the heater 1 than the heater line 11 is. The heater line 11 is provided with three thermal conductors 31, while the heater lines 14 are each provided with a thermal conductor 32 and a thermal conductor 33. Each of the thermal conductors 31 consists of a pair of heat blocks 3 provided on both sides of the heater line 11. Likewise, the thermal conductors 32 and 33 each consist of a pair of heat blocks 3 provided on both sides of the heater line 14.

The cell parts 2 are positioned while being sandwiched between two thermal conductors. For instance, five of the cell parts 2 are arranged between the thermal conductors 33 and 32 in a direction in which the thermal conductors extend. Accordingly, the cell parts 2 are applied with heat from both sides thereof.

Assuming that the heater line 11 is a first heater line, the heater lines 14 are second heater lines, the thermal conductors 31 are first thermal conductors, and the thermal conductors 32 and 33 are second thermal conductors, the above descriptions are understood as follows. That is, the heater 1 includes the first heater line 11, the second heater lines 14, the first thermal conductors 31 and the second thermal conductors 32 and 33. In addition, the first heater line 11 is provided with the plurality of first thermal conductors 31, and the second heater lines 14 are provided with the plurality of second thermal conducting parts 32 and 33.

According to the heater 1 described above, where a thermal conductor is divided to be provided for the heater lines, the weight and size of the heater are reduced further than when a single-piece thermal conductor is provided.

The first heater line 11 and the second heater lines 14 can be controlled to different temperatures in the heater 1 described above. At this time, temperatures near the thermal conductors can be varied for each heater, which allows concurrent culture and so on of microorganisms or cells under different conditions.

### 3. Third Preferred Embodiment

In a third preferred embodiment, an overall temperature of the heater 1 is made uniform. For instance, an overall temperature of the heater 1 shown in Fig. 5 can be made uniform as described below.

In addition to the descriptions given in the second preferred embodiment, the thermal conductors 31, 32 and 33 shown in Fig. 5 have a similar structure of the heat blocks 3 and are thus almost equal in thermal capacity. It is therefore assumed that thermal conductors provided for the same heater line, the thermal conductors 32 and 33 provided for the heater line 14, for example, are almost equal in temperature. Accordingly, by providing a thermometer for one thermal conductor of a plurality of thermal conductors provided for the same heater, temperatures of the other thermal conductors are estimated. The thermometer can be provided in an arbitrary position on the thermal conductor. In Fig. 5, as for the heater line 11, a thermometer 41 is provided in a center position on the centrally positioned thermal conductor 31 of the three thermal conductors 31. As for each of the heater lines 14, a thermometer 44 is provided in a center position on the thermal conductor 32.

The heater lines 11 and 14 are controlled to make the respective values of the thermometers 41 and 44 almost equal, so that the respective temperatures near the thermal conductors 31, 32 and 33 provided for the heater lines 11 and 14 can be made almost equal. In short, the overall temperature of the heater 1 can be made uniform. The result is that the temperatures inside the plurality of cell parts 2 can be made uniform as well.

The weight and size of the heater 1 were reduced by dividing a thermal conductor in the second preferred embodiment. Further reductions in weight and size of the heater 1 are desirable, however, in order to accomplish movement and so on of the temperature control device as easily as possible.

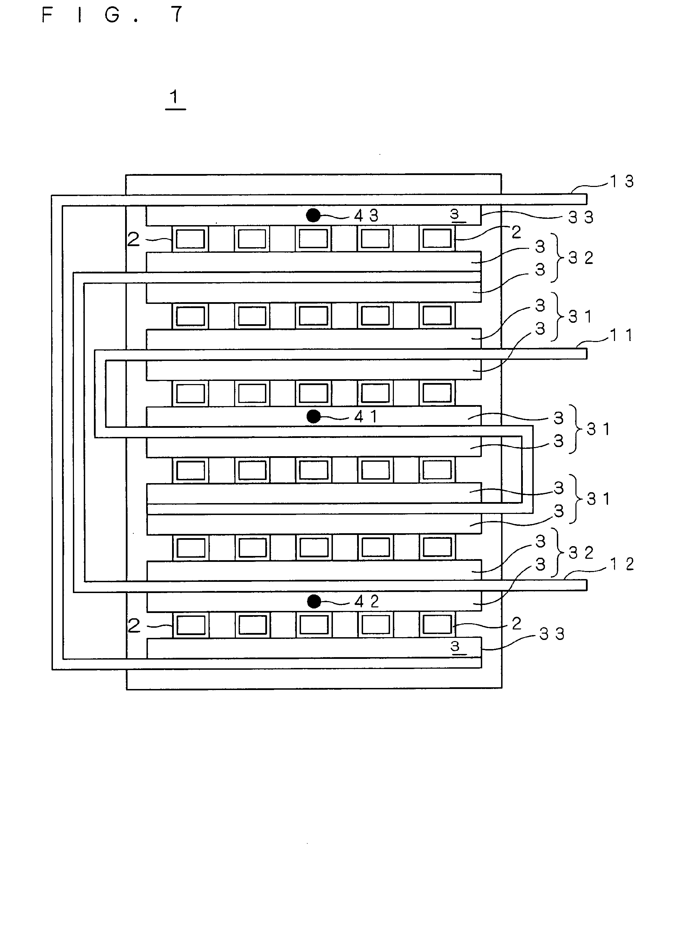

In a heater 1 shown in Fig. 6, the heat blocks 3 positioned nearest the outer edges in the heater 1 shown in Fig. 5 are removed. The weight and size of the heater 1 are therefore reduced.

However, the thermal conductor 32 and the thermal conductor 33 include different numbers of heat blocks 3, and are thus different in thermal capacity. For this reason, thermal conductors provided for the same heater line, namely, the thermal conductors 32 and 33 provided for the heater line 14, are different in temperature. Accordingly, the overall temperature of the heater 1 may become nonuniform.

In view of the above, a heater 1 shown in Fig. 7 can be employed. Elements of the heater 1 shown in Fig. 7 that are identical to those shown in Fig. 5 are denoted by the same reference numerals.

The heater 1 includes heater lines 11, 12 and 13. The heater line 13 is provided on an outer edge side of the heater 1 than the heater lines 11 and 12 are. The heater line 12 is provided with two thermal conductors 32 each of which consists of a pair of heat blocks 3. The heater line 13 is provided with two thermal conductors 33. Each of the thermal conductors 33 consists of one heat block 3 provided on the side of the heater lines 11 and 12, respectively.

The thermal conductors 31, 32 and the thermal conductor 33, consisting of the heat blocks 3, have different structures, and are thus different in thermal capacity. Yet the thermal conductors 31 and 32, or the thermal conductors 33 are almost equal in thermal capacity. That is, each of the heater lines is provided only with thermal conductors that are equal in thermal capacity. A thermometer 42 is provided for one of the thermal conductors 32, and a thermometer 43 is provided for one of the thermal conductors 33. The thermometers 42 and 43 can be provided in arbitrary positions on the thermal conductors. In Fig. 7, as for the heater line 12, the thermometer 42 is provided in a center position on one of the thermal conductors 32. As for the heater lines 13, the thermometer 43 is provided in a center position on one of the thermal conductors 33.

Assuming that the heater line 11 (12) is a first heater line, the heater line 13 is a second heater line, the thermal conductors 31 (32) are first thermal conductors, the thermal conductors 33 are second thermal conductors, the thermometer 41 (42) is a first thermometer, and the thermometer 43 is a second thermometer, the above descriptions are understood as follows.

That is, the heater 1 includes the first heater line 11 (12), the second heater line 13, the first thermal conductors 31 (32), the second thermal conductors 33, the first thermometer 41 (42), and the second thermometer 43. The plurality of the first thermal conductors 31 (32) are provided for the first heater line 11 (12). The plurality of the second thermal conductors 33 are provided for the second heater line 13. The first thermometer 41 (42) is provided for one of the first thermal conductors 31 (32). The second thermometer 43 is provided for one of the second thermal conductors 33. The first thermal conductors 31 (32) are equal in thermal capacity, and the second thermal capacitors 33 are also equal in thermal capacity. The first thermal conductors 31 (32) and the second thermal conductors 33 are different from each other in thermal capacity.

According to the heater 1 described above, the first thermal conductors 31 (32) and the second thermal conductors 33 are different in thermal capacity. This enhances a degree of freedom to arrange the first heater line 11 (12) and the second heater line 13 to heat the plurality of cell parts 2 uniformly. For instance, the first heater lines 11 and 12 can be made into one first heater line provided with the thermal conductors 31 and 32.

On the other hand, as the first thermal conductors 31 (32) are equal in thermal capacity, it is assumed that a temperature of the first thermal conductor 31 (32) measured by the first thermometer 41 (42) is almost equal to a temperature of the first thermal conductor 31 (32) not provided with the first thermometer 41 (42). Likewise, since the second thermal conductors 33 are equal in thermal capacity, a similar assumption can be made about the thermal conductor 33 not provided with the second thermometer 43.

Accordingly, even when the first thermal conductors 31 (32) are different from the second thermal conductors 33 in temperature, the first heater line 11 (12) and the second heater line 13 are controlled by using the first thermometer 41 (42) and the second thermometer 43, so that the temperatures near the first thermal conductors 31 (32) and the temperatures near the second thermal conductors 33 can be made almost equal. In short, the overall temperature of the heater can be made almost uniform, making temperature distribution inside the plurality of cell parts 2 uniform. The result is that the temperature to which a pace of culture and so on of microorganisms or cells is sensitive can be controlled accurately.

Moreover, the number of heat blocks 3 can be reduced while making the temperature distribution in the heater uniform. The weight and size of the heater 1 are therefore reduced.

In any of the preferred embodiments described above, the temperature control device can include a censor for each of the cell parts 2. The sensors measure a measurement value, such as oxygen concentration, that varies depending on metabolism of the microorganisms or cells. By measuring the measurement value, the number of microorganisms or cells is estimated.

More specifically, a culture medium is held inside the cell parts together with the microorganisms or cells, and oxygen concentration in the culture medium is measured based on a current flowing through the culture medium. The oxygen concentration in the culture medium varies with a change in the number of the microorganisms or cells in the culture medium. Accordingly, the number of microorganisms or cells is estimated by continuously measuring the current flowing through the culture medium.

In the temperature control device described above, the intra-cell temperature set to 35°C is suitable for viable bacteria inspection. The intra-cell temperatures set to 27°C, 30°C and 42°C are suitable for mold inspection, yeast inspection, and Escherichia coli inspection, respectively.

While the invention has been shown and described in detail, the foregoing description is in all aspects illustrative and not restrictive. It is therefore understood that numerous modifications and variations can be devised without departing from the scope of the invention.

## Claims

1. A temperature control device comprising:
a plurality of cell parts (2) holding microorganisms or cells; and
a heater (1) and a cooling unit (7) making control of temperatures inside said cell parts,
wherein said control is corrected by using an ambient temperature (T1).

2. The temperature control device according to claim 1, wherein said heater (1) comprises:
a first heater line (11; 11, 12) and a second heater line (14; 13);
a plurality of first thermal conductors (31; 31, 32) provided for said first heater line; and
a plurality of second thermal conductors (32, 33; 33) provided for said second heater line.

3. The temperature control device according to claim 1, wherein said heater (1) comprises:
a first heater line (11) and a second heater line (14);
a plurality of first thermal conductors (31) provided for said first heater line; and
a plurality of second thermal conductors (32, 33) provided for said second heater line,
wherein said first thermal conductor and said second thermal conductor are controlled to different temperatures from each other.

4. The temperature control device according to claim 1, wherein said heater (1) comprises:
a first heater line (11, 12) and a second heater line (13);
a plurality of first thermal conductors (31, 32) provided for said first heater line;
a plurality of second thermal conductors (33) provided for said second heater line;
a first thermometer (41, 42) provided for one of said first thermal conductors; and
a second thermometer (43) provided for one of said second thermal conductors,
said first thermal conductors being equal in thermal capacity,
said second thermal conductors being equal in thermal capacity, and
said first thermal conductors and said second thermal conductors being different from each other in thermal capacity.

5. The temperature control device according to claim 1, further comprising:
a thermometer (45) measuring an ambient temperature (T1);
a storage unit (5) storing calibration data; and
a control unit (6) setting a target value (T0) for said temperatures inside said cell parts, and controlling said heater (1) and said cooling unit (7) with a second target value (T2) that is obtained based on said target value (T0) and said calibration data in accordance with said ambient temperature.

6. The temperature control device according to claim 5, wherein said heater (1) comprises:
a first heater line (11; 11, 12) and a second heater line (14; 13);
a plurality of first thermal conductors (31; 31, 32) provided for said first heater line; and
a plurality of second thermal conductors (32, 33; 33) provided for said second heater line.

7. The temperature control device according to claim 5, wherein said heater (1) comprises:
a first heater line (11) and a second heater line (14);
a plurality of first thermal conductors (31) provided for said first heater line; and
a plurality of second thermal conductors (32, 33) provided for said second heater line,
wherein said first thermal conductor and said second thermal conductor are controlled to different temperatures from each other.

8. The temperature control device according to claim 5, wherein said heater (1) comprises:
a first heater line (11, 12) and a second heater line (13);
a plurality of first thermal conductors (31, 32) provided for said first heater line;
a plurality of second thermal conductors (33) provided for said second heater line;
a first thermometer (41, 42) provided for one of said first thermal conductors; and
a second thermometer (43) provided for one of said second thermal conductors,
said first thermal conductors being equal in thermal capacity,
said second thermal conductors being equal in thermal capacity, and
said first thermal conductors and said second thermal conductors being different from each other in thermal capacity.

9. The temperature control device according to claim 1, further comprising:
a thermometer (45) measuring an ambient temperature (T1);
a control unit (6) setting a target value (T0) for said temperatures inside said cell parts; and
a calculation unit, wherein
said calculation unit calculates a second target value (T2) from said ambient temperature and said target value (T0), and
said control unit controls said heater (1) and said cooling unit (7) with said second target value (T2).

10. The temperature control device according to claim 9, wherein said heater (1) comprises:
a first heater line (11; 11, 12) and a second heater line (14; 13);
a plurality of first thermal conductors (31; 31, 32) provided for said first heater line; and
a plurality of second thermal conductors (32, 33; 33) provided for said second heater line.

11. The temperature control device according to claim 9, wherein said heater (1) comprises:
a first heater line (11) and a second heater line (14);
a plurality of first thermal conductors (31) provided for said first heater line; and
a plurality of second thermal conductors (32, 33) provided for said second heater line,
wherein said first thermal conductor and said second thermal conductor are controlled to different temperatures from each other.

12. The temperature control device according to claim 9, wherein said heater (1) comprises:
a first heater line (11, 12) and a second heater line (13);
a plurality of first thermal conductors (31, 32) provided for said first heater line;
a plurality of second thermal conductors (33) provided for said second heater line;
a first thermometer (41, 42) provided for one of said first thermal conductors; and
a second thermometer (43) provided for one of said second thermal conductors,
said first thermal conductors being equal in thermal capacity,
said second thermal conductors being equal in thermal capacity, and
said first thermal conductors and said second thermal conductors being different from each other in thermal capacity.

13. The temperature control device according to claim 12, wherein
said second heater line (13) is provided on an outer edge side of said heater than said first heater line (11, 12) is,
each of said first thermal conductors (31, 32) includes a pair of heat blocks (3) provided on both sides of said first heater line, and
each of said second thermal conductors (33) includes one heat block (3) provided for said second heater line on the side of said first heater line.

14. The temperature control device according to any one of claims 1 to 13, further comprising:
a sensor for each of said cell parts (2), said sensor measuring a measurement value that varies depending on metabolism of said microorganisms or cells.
